# EUROPEAN PATENT APPLICATION

(11) **EP 2 045 326 A1**
(43) Date of publication of application: **08.04.2009**
(21) Application number: 07019557.3
(22) Date of filing: 05.10.2007
(51) Int. Cl.: C12N 15/10, G01N 33/68

(54) **Method for identifying the interaction partner of an active agent**

(71) Applicant: Bayer CropScience AG, 40789 Monheim (DE)
(72) Inventor: Reif, Hans Jörg, Dr., 50939 Köln (DE)

(57) **Abstract**

The present invention is related to a method for identifying an interaction partner of an active agent, comprising the following steps:
(a) providing an element library comprising a plurality of elements,
(b) contacting the element library with a preparation comprising an interaction partner under conditions allowing for formation of a complex consisting of a binding element of the element library binding to the interaction partner and the interaction partner, whereupon a complex comprising the binding element and the interaction partner is formed,
(c) removing non-binding elements of the element library from the complex formed in step (b),
(d) contacting the complex formed in step (b) with the active agent, whereby the binding element is released from said complex,
(e) contacting the binding element released in step (d) with a preparation comprising the interaction partner, whereupon a complex is formed comprising the binding element released in step (d) and the interaction partner, and
(f) characterising the interaction partner.

## Description

The present invention is related to a method for identifying the interaction partner of an active agent, an interaction partner identifiable by such a method and a method for determining the mode of action of an active agent.

The era of structural genomics has seen the completion of genome projects providing researchers with exhaustive sequence information on a number of organisms, not only including man (The sequence of the human genome. The Celera Genomics Sequencing Team, Science, 2001: Vol 291:1304-1351, International Human Genome Sequencing Consortium. Finishing the euchromatic sequence of the human genome, Nature, 2004: Vol 431(7011):927-930), and model organisms such as mouse (*Mus musculus*) (Mouse Genome Sequencing Consortium. Initial sequencing and comparative analysis of the mouse genome. Nature, 2002, Vol 420:520-562), rat (*Rattus rattus*) (Rat Genome Sequencing Project Consortium. Genome sequence of the Brown Norway rat yields insights into mammalian evolution. Nature, 2004: Vol 428(6982):493-521), dog (*Canis lupus familiaris*) (Lindblad-Toh et al Nature, 2005: Vol 438:803-819) and wall cress (*Arabidopsis thaliana*) (Analysis of the genome sequence of the flowering plant Arabidopsis thaliana. Nature, 2000: Vol 408:796 - 815 special), but also organisms of paramount biotechnological and agricultural importance, for instance *Escherichia coli* (Frederick R. Blattner et al., Science, 1997: Vol 277: 1453 - 1462), rice (*Oryza sativa ssp. Japonica*) (International Rice Genome Sequencing Project: The map-based sequence of the rice genome. Nature, 2005: Vol 436: 793-800), and yeast (*Saccharomyces cerevisiae*) (The yeast genome directory, Nature, 2004: Vol 387: 66325 S).

While the generation of sequence data, together with development of increasingly powerful bioinformatic and analytical tools (for review: Minoru Kanehisa & Peer Bork: Bioinformatics in the post-sequence era. Nature Genetics, 2003: Vol 33: 305 - 310; Spannagl et al., Nucleic Acids Res. 2007: Vol 35(Database Issue): D834-D840: MIPSPlantsDB - Plant database resource for integrative and comparative plant genome research; Stuempflen V, Mewes HW: BIOspektrum 2007: Vol 3: 281-283, Neue Informationstechnologien zur Nutzung biologischer Daten) has revolutionised proteomic investigations (Mewes HW, et al., MIPS: Analysis and annotation of proteins from whole genomes in 2005. Nucl. Acids Res., 2006: Vol 34(suppl_1): D169-D172; Schmidt A., et al.: A novel strategy for quantitative proteomics using isotope-coded protein labels. Proteomics, 2005: Vol 1: 4-15; Niklas Gustavsson: A proteomic method for the analysis of changes in protein concentrations in response to systemic perturbations using metabolic incorporation of stable isotopes and mass spectrometry. Proteomics, 2004: Vol 5: 3563 - 3570; Daniel C. Liebler: Introduction to Proteomics: Tools for the New Biology, 2007 Humana Press, Totowa, NJ, USA), limited functional information on newly identified proteins, often solely a prediction based on sequence alignments, has been supplied. At the same time, links between known active agents and their interaction partners, remain elusive, which corroborates the need to devise new ways to establish functional relationships which may lead to the identification and mapping of promising interaction partners, and to a better understanding of the mode of action of the respective biologically active substance which is a key requirement for the rational design of novel biotechnologically, agriculturally, and medically useful compounds.

A range of routine techniques have been established such as affinity chromatography (Godl K. et al. (2003), Proc Natl Acad Sci U S A, 100(26):15434-9; Daub H. et al. (2004), Assay Drug Dev Technol., 2(2):215-24; Brehmer D. et al. (2005), Cancer Res., 65(2):379-82) or yeast three hybrid systems (Drees B.L. (1999), CurrOpin Chem Biol.,3(1):64-70; Kley N. (2004), Chem Biol.,11(5):599-608; Becker F. et al. (2004), Chem Biol.,11(2):211-23) in order to isolate interaction partners of a known molecule that elicits a biological phenotype, most often relying on the immobilisation of the active agent and its subsequent use as a ligand to capture the elusive target, which is usually present in a pool comprising thousands of candidate interaction partners. However, in order for the active agent to be immobilised efficiently, a chemical modification needs to be introduced that allows for the covalent attachment of the active agent to a support, but does not hamper with its ability to bind to the interaction partner in a physiologically relevant manner. Such a modification is often straightforward to devise if biological macromolecules, for example proteins and nucleic acids, are to be immobilised, as a range of standard methods, for examples, amine and thiol coupling, biotinylation, the use of GST- or His-tags and the like are available (Hermanson, G.T., Bioconjugate Techniques, 1996, Academic Press, San Diego; Blank, M. et al., J Biol Chem. 2006, 276(19): 16464-8; Porschewski, P. et al., J Biomol Screen. 2006, 11(7):773-81; Current Protocols in Protein Science, 2007, last supplemented May 2007, Coligan J. E., Dunn B.M., Speicher D.W., Wingfield P.T., eds., John Wiley and Sons Inc. New York). In this context, it should be noted that only a small fraction of the atoms associated with the macromolecule are actually involved in the binding event, *i*.*e*. the interaction; hence, many exposed, functional groups are available, or may be incorporated through genetic engineering, that can be modified without impairing the capability of the macromolecule to bind to the interaction partner. By contrast, a high proportion of the atoms of a molecule are critical if a small molecule such as a low molecular weight active agent is used to capture an interaction partner, increasing the risk that a modification will interfere with critical interactions between the small molecule and the interaction partner, which may result in a partial or complete loss of affinity. If the structure of the interaction partner has been solved at reasonably high resolution, it may be possible to use molecular modelling techniques to design promising modified active agents as ligands; however, in the absence of extensive structural information, which, if an unknown interaction partner is to be captured, is not available, the rational design of a non-interfering modification for the purpose of immobilisation is beyond technical feasibility.

An alternative strategy involves radiolabelled active agents, which can be used to mark fractionated and/or purified interaction partners, but this is not only associated with hazardous experiments involving radiation, which can be carried out by trained staff using designated facilities only, but does not actually provide a means to isolate and vigorously identify interaction partners (Bylund D.B. and Murrin L.C. (2000), Life Sci., 67(24):2897-911, Wu S. and Liu B. (2005), BioDrugs., 19(6):383-92). A multitude of techniques, such as the use of NMR spectroscopy, fluorescence spectroscopy, isothermal calorimetry, mass spectrometry and the like, have been established to confirm complex formation between an active agent and an interaction partner (Methods in Enzymology, Fluorescence Spectroscopy (1997), Vol. 278, Brand L. and Johnson M.L. Eds., Academic Press, San Diego; Weber P.C. and Salemme F.R., (2003) Curr Opin Struct Biol., 13(1):115-21; Wishart D. (2005), Curr Pharm Biotechnol., 6(2):105-20; Methods in Enzymology: Biological Mass Spectrometry (2005), Vol. 402, Burlingame A.L., ed., Academic Press, San Diego), but fail to identify said interaction partner. In some cases, bioinformatic analyses can be used to screen genomic data for potential interaction partners, but this type of analysis is always associated with a high degree of uncertainty, and extensive experimentation is required to verify interaction partner hits.

Therefore, there is high demand for methods that are suitable to identify interaction partners using molecules that, on the one hand, have the ability to act as high-affinity ligands to a specific binding site, and are, on the other hand, sufficiently versatile to be chemically modified and linked to a support without detrimentally affecting said binding activity. Ideally, such methods should yield ligands that are stable under physiologically relevant conditions and accessible through chemical synthesis or biological amplification to allow for their subsequent use as capture agents to identify the interaction partner(s) of interest.

Thus, a problem underlying the present invention is to provide a method for the identification of an interaction partner to a given active agent which is preferably biologically active. A further problem underlying the present invention is to elucidate the mode of action of such an active agent.

The problem underlying the present invention is solved in a first aspect by a method for identifying an interaction partner of an active agent, comprising the following steps:
(a) providing an element library comprising a plurality of elements,
(b) contacting the element library with a preparation comprising an interaction partner under conditions allowing for formation of a complex consisting of a binding element of the element library binding to the interaction partner and the interaction partner, whereupon a complex comprising the binding element and the interaction partner is formed,
(c) removing non-binding elements of the element library from the complex formed in step (b),
(d) contacting the complex formed in step (b) with the active agent, whereby the binding element is released from said complex,
(e) contacting the binding element released in step (d) with a preparation comprising the interaction partner, whereupon a complex is formed comprising the binding element released in step (d) and the interaction partner, and
(f) characterising the interaction partner.

In an embodiment, the binding element released in step (d) is isolated in a step d'.

In an embodiment, the binding element released in step (d) is amplified in a step d".

In an embodiment, steps (a) to (d) and, optionally, the isolation (d') and/or amplification (d") of the element released in step (d) are repeated.

In an embodiment, the interaction partner is released from the complex formed in step e) prior to step (f).

In an embodiment, step (d) comprises the unspecific or specific elution of the binding element.

In an embodiment, the method is for identifying two or more interaction partners.

In an embodiment, the elements are ribonucleic acids.

In an embodiment, the elements are deoxyribonucleic acids.

In an embodiment, the elements are peptides.

In an embodiment, any of the preparations comprising the interaction partner is selected from the group comprising a proteome of an organism or a fraction thereof, a cellular extract of an organism, an extracellular extract of an organism, a subcellular extract of an organism and an extract of a whole organism.

In an embodiment, the binding element released in step (d) is immobilised on a support prior to step (e).

In an embodiment, the interaction partner is characterised by a technique selected from the group comprising mass spectrometry, sequencing techniques, binding assays, enzyme activity assays, electrophoresis and immunological methods.

In an embodiment, the active agent is selected from the group consisting of pharmaceutically active agents, agrochemically active agents, therapeutically active agents, diagnostically active agents, synergists, veterinary pharmaceuticals, animal and human nutrition-enhancing compounds comprising nutraceuticals, and plant protection agents, and a respective precursor of each thereof.

In a preferred embodiment, the plant protection agent is selected from the group consisting of herbicides, fungicides, active agents against oomycetes, nematocides, insecticides, safeners, and combinations of safeners and plant protection agents.

In an embodiment, the veterinary pharmaceuticals are selected from the group consisting of antiviral compounds, antibiotics and anti-parasite drugs.

In an embodiment, the interaction partner is selected from the group consisting of peptides, polypeptides, proteins, nucleic acids, lipids, and carbohydrates, or any combination thereof.

In an embodiment, the interaction partner is a protein, whereby the protein is preferably selected from the group consisting of enzymes, receptors and transcription factors.

In an embodiment, the various elements of the element library comprise a variable moiety and a constant moiety, whereby the constant moiety is the same for the majority, preferably all of the elements of the element library.

In a preferred embodiment, the element library consists of nucleic acids and the constant moiety consists of a 3'-terminal or 5'-terminal stretch of nucleotides.

In a further preferred embodiment, the constant moiety consists of from about 10 to about 30 nucleotides.

In an embodiment, the element library consists of nucleic acids, whereby the variable moiety consists of from about 8 to 120 nucleotides.

In a preferred embodiment, the element library consists of peptides and the constant moiety consists of an N-terminal or C-terminal stretch of amino acids.

In an embodiment, the step of removing non-binding elements as of step (c), the step of isolating the binding elements and/or the step of amplifying the elements make use of the constant moiety of the elements of the element library.

In an embodiment, the elements are nucleic acids and wherein the amplification is performed by reverse transcription, PCR, RNA transcription or isothermal amplification (Romano J.W. et al. (1997), Immunol Invest., 26(1-2):15-28; Kievits T. (1991), J Virol Methods., 35(3):273-86; Methods in Enzymology: Combinatorial Chemistry (1996), Vol 267, Abelson J.N. and Simon M.I., eds. Academic Press, San Diego; Sambrook J., Russel D.W. (2001), Molecular Cloning: A Laboratory Manual 3rd edition, Cold Spring Harbour Laboratory Press, Cold Spring Harbour, New York; Current Protocols in Nucleic Acid Chemistry (2007), last supplemented March 2007, Beaucage S.L., Bergstrom D. E., Herdewijn P., Matusda A., eds., John Wiley and Sons Inc. New York).

In an embodiment, the elements are peptides expressed by a phage display technique (Phage Display of Peptides and Proteins: A Laboratory Manual (1996), Kay B.K., Winter J., McCafferty J. eds., Academic Press, San Diego) and the amplification is performed by amplifying the phages expressing the peptides.

In an embodiment, the organism is selected from the group consisting of humans, prokaryotes, fungi, animals and plants.

In an embodiment, the immobilisation of the binding element is mediated by the constant moiety of the element.

The problem underlying the present invention is solved in a second aspect by an interaction partner identifiable by a method according to the first aspect.

The problem underlying the present invention is solved in a third aspect by a method for determining the mode of action of an active agent, comprising the steps of the method according to the first aspect.

The present invention is based on the surprising finding that elements can be isolated from an element library using an uncharacterised active agent to identify the interaction partner to said active agent, even if the functional relationship between the two can be unknown.

It is within the present invention that not only one element of the element library is binding to the interaction, but two or more elements of the element library. Accordingly, in an embodiment, also these two or more binding elements are released from the complex, optionally isolated and/or amplified.

In a preferred embodiment, the term "interaction partner", as used herein, refers to a molecule that interacts with another molecule. According to the present invention, such another molecule is an active agent. However, it will be acknowledged by a person skilled in the art that the active agent is typically different from the molecule with which the interaction partner, which is preferably a molecule present in a biological system such as an organism as defined herein, is interacting in such a biological system. Such an interaction is mediated by physical forces, for instance electrostatic attraction, hydrogen bonds or van der Waals bonds, resulting in binding of the interaction partner to the other molecule. The interaction can be of transient nature, resulting in reversible binding, or can be permanent, for example through formation of a covalent bond. A particularly preferred interaction partner, as expected to be identified using the method according to the present invention, could be a protein in the cytosol of a cell. Another particularly preferred interaction partner could be a membrane protein; however, the interaction partner need not necessarily to be a protein, but can be a nucleic acid, a lipid, a carbohydrate, a combination of any of the afore-mentioned compound classes, or any other kind of molecule. In the context of the present invention, the other molecule, which interacts with the interaction partner, shall mean the active agent, and the interaction partner features a binding site for the active agent, with the binding event being causatively linked to the biological activity of the active agent. In any event, no information on the interaction partner to be identified is required to successfully use the method according to the present invention.

In an embodiment of the present invention, the interaction partner is not only bound by the active agent, but is inhibited by the active agent either by competitive binding or by allosteric binding.

It is also within the present invention that the binding element is eluted in either a competitive manner or an allosteric manner. Such inhibition is preferably detected or confirmed in an embodiment of the method according to the present invention where as a further step or sequence of steps the mode of action of the active agent is determined. Alternatively, the function of the interaction partner is already known. The respective procedures are known to a person skilled in the art and, preferably, comprise functional tests. Such functional tests are preferably based on the biological activity of the respective interaction partner. If, for example, the interaction partner is an enzyme, such functional assay will be an enzymatic assay, if the interaction partner is a protein-binding protein, such functional assay could be a coprecipitation assay or a yeast two-hybrid assay and if the interaction partner is a nucleic acid-binding protein, such functional assay could be a gel retardation assay (Dixon M. and Webb E.C. (1966), Enzymes 2nd ed. (5th impression), Longmans, Green and Co LTD, London; Sambrook J., Russel D.W. (2001) Molecular Cloning: A Laboratory Manual 3rd edition, Cold Spring Harbour Laboratory Press, Cold Spring Harbour, New York; Current Protocols in Nucleic Acid Chemistry (2007), last supplemented March 2007, Beaucage S.L., Bergstrom D. E., Herdewijn P., Matusda A., eds., John Wiley and Sons Inc. New York, Current Protocols in Molecular Biology (2007), last supplemented April 2007, Ausubel F.M., Brent R., Kingston R.E., Moore D.D., Smith J.A., Struhl K., Medical School eds., John Wiley and Sons Inc. New York).

In a preferred embodiment, the term "active agent", as used herein, refers to a given molecule that elicits a phenotype when administered to a biological system. For example, an active agent could be a small molecule capable of entering the cell and functioning through interaction with one or more cellular molecules or structures, but active agents could as well include any type of biologically active molecule including macromolecules such as proteins.

In a preferred embodiment, two or more interaction partners corresponding to or for a given active agent can be identified. Moreover, in line with interaction partner mapping or profiling studies, in a preferred embodiment of the invention, interaction partners to a given active agent can be identified with respect to specific tissues, structures, organelles or cellular compartments of an organism or the like.

In a preferred embodiment, the term "uncharacterised active agent", as used herein, shall mean that the active agent need not be functionally and/or structurally characterised; neither is any information on the interaction between the active agent and the interaction partner or the interaction partners required to qualify for the performance of the method of the present invention.

In a preferred embodiment, the term "biological system", as used herein, refers to any type of organism or mixture of organism, as well as fractions or preparations made of organisms.

In a preferred embodiment, the term "element", as used herein, refers to a molecule that does or does not bind to an interaction partner or another molecule of interest. In a preferred embodiment, the element is a deoxynucleic acid. In another preferred embodiment, the element is a ribonucleic acid. In another preferred embodiment, the element is a peptide. In another preferred embodiment, the element is resistant to cellular degradation, for example through the incorporation of a stability conferring moiety such as one or several chemically modified nucleotide(s) into a nucleic acid.

In a preferred embodiment, the term "nucleic acid", as used herein, refers to a polymer comprising at least two nucleotide units attached to each other *via* phosphodiester bonds. Nucleotide units typically include a sugar moiety, for example ribose or deoxyribose, attached to a base, for instance adenine, guanine, thymine, cytosine or uracil and chemically modified forms thereof, although the scope of the invention includes alternative sugar moiety, bases and backbones such as the ones incorporated through the use of artificial nucleotide in chemical syntheses including phosphothioates, 2' fluoro deoxy, 2' methoxy or 2' amino deoxy nucleotides as, e. g., described in Pagratis N.C. et al. (1997), Nat Biotechnol. 15(1):68-73; Eaton B. E. et al. (1997), Bioorg Med Chem. 5(6):1087-96; Eaton, B. E. (1997), Curr Opin Chem Biol. 1(1):10-6 and Ruckman J. et al. (1998), Biol Chem. 273(32):20556-67. In one embodiment of the present invention, the nucleic acid is deoxyribonucleic acid, in a more preferred embodiment of the present invention, the nucleic acid is a ribonucleic acid.

In another preferred embodiment of the present invention, the nucleic acid is an aptamer. Aptamers, as referred herein, are D-nucleic acids which are either single stranded or double stranded and which specifically interact with an interaction partner. The manufacture or selection of aptamers is, *e*. *g*., described in European patent EP 0 533 838 or in Ellington, A. D., Szostak, J. W. (1990) Nature 346, 818-822, Tuerk, C., Gold, L. (1990) Science 249, 505-510, Methods in Enzymology: Combinatorial Chemistry (1996), Vol 267, Abelson J.N. and Simon M.I., eds. Academic Press, San Diego; Current Protocols in Nucleic Acid Chemistry, (2007), last supplemented March 2007, Beaucage S.L., Bergstrom D. E., Herdewijn P., Matusda A., eds., John Wiley and Sons Inc. New York). Basically the following steps are performed. First, a mixture of nucleic acids, *i.e*. potential aptamers, is provided whereby each nucleic acid typically comprises a segment of several, preferably at least eight subsequent randomised nucleotides. This mixture is subsequently contacted with the interaction partner whereby the nucleic acid(s) bind to the interaction partner, such as based on an increased affinity towards the interaction partner or with a bigger force thereto, compared to the candidate mixture. The binding nucleic acid(s) are/is subsequently separated from the remainder of the mixture. Optionally, the thus obtained nucleic acid(s) is amplified using, *e*.*g*. polymerase chain reaction in the case of DNA aptamers and reverse transcription, polymerase chain reaction and RNA transcription in the case of RNA aptamers. These steps may be repeated several times giving at the end a mixture having an increased ratio of nucleic acids specifically binding to the target from which the final binding nucleic acid is then optionally selected. These specifically binding nucleic acid(s) are referred to as aptamers. It is obvious that at any stage of the method for the generation or identification of the aptamers samples of the mixture of individual nucleic acids may be taken to determine the sequence thereof using standard techniques. It is within the present invention that the aptamers may be stabilized such as, *e*. *g*., by introducing defined chemical groups which are known to the one skilled in the art of generating aptamers. Such modification may, for example, reside in the introduction of an amino, fluoro or methoxy group at the 2'-position of the sugar moiety of the nucleotides. Aptamers are currently used as therapeutical agents. For example an anti-VEGF aptamer (Pegaptanib, Macugen) has been approved for the treatment of wet age-related macular degeneration (Kourls H. and Schiller D.S (2006)., Clin Ther. 28(1):36-44). However, it is also within the present invention that the thus selected or generated aptamers may be used for target validation and/or as lead substance for the development of biologically active substances such as herbicides, fungicides, nematocides or medicaments (P. Burgstaller at al. (2002), Curr Opin Drug Discov Devel. 5(5):690-700; M. Blank and M. Blind (2005), Curr Opin Chem Biol., 9(4):336-42), preferably of biologically active substances based on small molecules. This is actually done by a competition assay or rational design whereby the specific interaction between the interaction partner and the aptamer is inhibited by a candidate drug whereby upon replacement of the aptamer from the complex of the interaction partner and aptamer it may be assumed that the respective drug candidate allows a specific inhibition of the interaction between the interaction partner and aptamer, and if the interaction is specific, said candidate drug will, at least in principle, be suitable to block the interaction partner and thus decrease its biological availability or activity in a respective system comprising such an interaction partner. The thus obtained small molecule may then be subjected to further derivatisation and modification to optimise its physical, chemical, biological and/or medical characteristics such as toxicity, specificity, biodegradability and bioavailability.

In another embodiment the nucleic acid is a spielgelmer. Spiegelmers are L-nucleic acids, which means that they are composed of L-nucleotides in contrast to aptamers which are composed of D-nucleotides. Spiegelmers are characterized by the fact that they have a very high stability in biological system and, comparable to aptamers, specifically interact with a target molecule equivalent to the interaction partner of the present application, against which they are directed. For the purpose of generating spiegelmers, a heterogonous population of D-nucleic acids is created and this population is contacted with the optical antipode of the target molecule. Subsequently, those D-nucleic acids are separated which do not interact with the optical antipode of the target molecule. However, those D-nucleic acids interacting with the optical antipode of the target molecule are separated, optionally determined and/or sequenced and subsequently the corresponding L-nucleic acids are synthesized based on the nucleic acid sequence information obtained from the D-nucleic acids. These L-nucleic acids which are identical in terms of sequence with the aforementioned D-nucleic acids interacting with the optical antipode of the target molecule, will specifically interact with the naturally occurring target molecule rather than with the optical antipode thereof. Similar to the method for the generation of aptamers, it is also possible to repeat the various steps several times and thus to enrich those nucleic acids specifically interacting with the optical antipode of the target molecule. The manufacture and generation, respectively, of spiegelmers is described in the international patent application WO 98/08856.

In a preferred embodiment, the term "peptide", as used herein, refers to a polymer comprising at least two amino acids covalently linked through peptide bonds. Amino acids typically include the natural proteinogenic amino acids, in particular glycine, alanine, valine, leucine, isoleucine, phenylalanine, tyrosine, tryptophan, cystein, methionine, serine, threonine, lysine, arginine, histidine, aspartate, glutamate, asparagine and glutamine, but also any other kind of amino acid that can be chemically or enzymatically synthesised. The term also includes polymers comprising amino acid derivatives including D-amino acids.

In a preferred embodiment, the terms "library" or "element library", as used herein, refer to a plurality of elements, comprising at least as little as two elements and, in a particularly preferred embodiment, more than 10¹⁵ different species. Such elements are usually chemically synthesised, although other methods such as the isolation from biological systems or the degradation of larger molecules could in principle be used for providing such elements. The individual elements of the library differ in terms of their chemical features, for example by comprising different nucleotide or peptide sequence elements in the case of nucleic acids and peptides, respectively. Preferably, the library used according to the present invention is sufficiently diverse to comprise at least one binding element that binds to the interaction partner to be identified, although the majority of molecules are typically non-binding molecules that do not form significant interactions with the interaction partner.

In a preferred embodiment, the term "preparation comprising the interaction partner", as used herein, refers to any kind of preparation that comprises the interaction partner and possibly, but not necessarily, other molecules. In an embodiment of the present invention, the preparation comprises the proteome of an organism or a mixture of organisms or fractions thereof. In another embodiment of the present invention, the preparation comprises a cellular extract. In another embodiment of the present invention, the preparation comprises an extracellular extract from an organism. In another embodiment of the present invention, the preparation comprises a subcellular extract. In a final embodiment of the present invention, the preparation comprises an extract derived from an organism.

In a preferred embodiment, the term "formation of a complex", as used herein, shall mean a direct physical or chemical association of two molecules, which is not a random event. Such an association requires the two molecules to approach one another geometrically, resulting in a contact which will exclude other molecules in the solution from making contact through the same contact site, referred to as binding site. A crucial feature of complex formation in the context of the present invention is that the association is reversible, meaning that the two molecules can subsequently dissociate or be separated as a result, in a preferred embodiment through the presence of alternative molecules that are able to associate with one of the two molecules in a thermodynamically more favourable manner, thus displacing one of the two molecules. This is in contrast to the association of the interaction partner and the active agent, which, in one embodiment, is irreversible, but may as well be reversible.

In a preferred embodiment, the term "removing" a molecule "from the complex", as used herein, refers to any kind of procedure the performance of which results in the absence of said molecule from the original reaction comprising the complex. The person skilled in the art is aware of a variety of suitable techniques such as the use of preparative ultracentrifugation, dialysis, chromatography, more specifically competitive chromatography and size-exclusion chromatography, preparative gel electrophoresis, filter retention (assays) and pull-down techniques including pull-down assays (Sambrook J., Russel D.W. (2001), supra; Current Protocols in Nucleic Acid Chemistry (2007), last supplemented March 2007, Beaucage S.L., Bergstrom D. E., Herdewijn P., Matusda A., eds., John Wiley and Sons Inc. New York; Current Protocols in Protein Science (2007), last supplemented May 2007, Coligan J. E., Dunn B.M., Speicher D.W., Wingfield P.T., eds., John Wiley and Sons Inc. New York).

The term "amplification", as used herein, refers to the multiplication of molecules, in particular nucleic acids. In an embodiment of the present invention, a suitable polymerase is used to synthesise new nucleic acid molecules using a smaller quantity of present nucleic acids as a template. A number of routine methods and suitable reagents that may be used to accomplish the amplification are well known to the person skilled in the art (Romano J.W. et al. (1997), supra; Kievits T.J., (1991), supra; Methods in Enzymology: Combinatorial Chemistry (1996), Vol 267, Abelson J.N. and Simon M.I., eds. Academic Press, San Diego; Sambrook J., Russel D.W. (2001), supra; Current Protocols in Nucleic Acid Chemistry (2007), last supplemented March 2007, Beaucage S.L., Bergstrom D.E., Herdewijn P., Matusda A., eds., John Wiley and Sons Inc. New York).

The term "characterising the interaction partner", as used herein, refers to any technique that allows researchers to establish the identity, or at least crucial features of an interaction partner to the known active agent. In an embodiment of the present invention, the optionally previously isolated interaction partner(s) is/are characterised using, for example C-terminal and N-terminal (Edman) sequencing as described in Current Protocols in Protein Science (2007), last supplemented May 2007, Coligan J. E., Dunn B.M., Speicher D.W., Wingfield P.T., eds., John Wiley and Sons Inc. New York., in the case of a protein being the interaction partner(s) or the Sanger Dideoxy method, PCR, reverse transcription and PCR or specific hybridization (Sambrook J., Russel D.W. (2001), supra; Current Protocols in Nucleic Acid Chemistry (2007), last supplemented March 2007, Beaucage S.L., Bergstrom D.E., Herdewijn P., Matusda A., eds., John Wiley and Sons Inc. New York; Current Protocols in Molecular Biology (2007), last supplemented April 2007, Ausubel F.M., Brent R., Kingston R.E., Moore D.D., Smith J.A., Struhl K., Medical School eds., John Wiley and Sons Inc. New York), in the case of a nucleic acid being the interaction partner(s), and/or bioinformatic tools, e.g. database analysis of sequence information on the target. In another embodiment of the present invention, the characterisation procedure comprises the use of a protease, for example trypsin, to digest a protein interaction partner(s) and/or the subsequent analysis of the protein of fragments thereof by mass spectrometry, peptide mass fingerprinting, MALDI-MS, or MALDI-TOF-MS (Mann M et al. (1993), Biol Mass Spectrom 22:338-345; Current Protocols in Protein Science (2007), last supplemented May 2007, Coligan J. E., Dunn B.M., Speicher D.W., Wingfield P.T., eds., John Wiley and Sons Inc. New York).

In another embodiment of the present invention, binding assays taking advantage of known, specific ligands such as antibodies for instance ELISA, Western blot or pull down assays are used to characterise the optionally previously isolated interaction partner (Sambrook J., Russel D.W. (2001), supra ; Current Protocols in Protein Science (2007), last supplemented May 2007, Coligan J. E., Dunn B.M., Speicher D.W., Wingfield P.T., eds., John Wiley and Sons Inc. New York). In another embodiment of the present invention, enzymatic activity assays are used to characterise the target (Dixon M. and Webb E.C. (1966), Enzymes 2nd ed. (5th impression), Longmans, Green and Co LTD, London). In another embodiment of the present invention, the characterisation of the target is accomplished through the use of electrophoretic techniques such as capillary electrophoresis (Current Protocols in Molecular Biology (2007), last supplemented April 2007, Ausubel F.M., Brent R., Kingston R.E., Moore D.D., Smith J.A., Struhl K., Medical School eds., John Wiley and Sons Inc. New York).

In another embodiment, the interaction partner is not a protein, and other standard techniques of organic analytical chemistry can be applied for identification such as, e.g. described in Encyclopedia of Analytical Chemistry, R.A. Meyers, ed. (2000) Wiley & Sons New York.

In a preferred embodiment, the term "specific elution", as used herein, refers to a procedure that leads to the removal of a significant amount of elements, in a most preferred embodiment all elements, belonging to one particular group of elements from complexes, while elements other than those belonging to said group are removed less efficiently or, in a most preferred embodiment, not removed at all. Typically, an excess of active agent molecules is used to dissociate from the interaction partner binding elements that bind to an or the active agent's binding site on an interaction partner, while binding elements binding to sites other than the active agent's binding site do not dissociate from the interaction partner.

In a preferred embodiment, the term "unspecific elution", as used herein refers to a procedure that leads to the removal of elements, in a most preferred embodiment all elements, from complexes, regardless of whether or not they belong to a particular group of elements. Typically, a sample comprising complexes comprising or consisting of interaction partners and binding elements is heated up to thermally dissociate all elements from interactions partners.

Plant protection agents as preferably used herein comprise, but are not limited to herbicides, insecticides, fungicides, nematicides, active agents against oomycetes, safeners and synergists as defined in "The Pesticide Manual", 14th edition, C.D.S. Tomlin, BCPC, British Crop Production Council, or "Modern Crop Protection Compounds", Eds W Krämer U Schirmer, 2007, Wiley-VCH Verlag.

In a preferred embodiment, the term "herbicide", as used herein, refers to a compound that detrimentally affects the growth, development and/or fertility of undesired plant growth.

In a preferred embodiment, the term "fungicide", as used herein, refers to a compound that detrimentally affects the growth, development and/or fertility of a fungus and/or its ability to multiply and/or infect. In a preferred embodiment, the fungicide kills said fungus. In a preferred embodiment, the fungicide can be used to treat humans, animals, plants and/or any other organism suffering from an fungal infection or to prevent them from getting or developing such infection.

In a preferred embodiment, the term "insecticide", as used herein, refers to a compound that detrimentally affects the growth, development, health and/or fertility of an insect and preferably kills said insect. In a preferred embodiment, the insecticide can be used to treat humans, animals, plants, plant growing areas, plant seeds and/or any other organism suffering from an insecticidal attack or infection or being at risk to get or develop such infection.

In a preferred embodiment, the term "nematicide", as used herein, refers to a compound that detrimentally affects the growth, development, fertility and/or health of a nematode and/or its ability to multiply and/or infect. In a preferred embodiment, the nematocide kills said nematode. In a preferred embodiment, the nematocide can be used to treat humans, animals, plants and/or any other organism suffering from an infection related to a nematode or to prevent them from getting or developing such infection.

In a preferred embodiment, the term "safener", as used herein, refers to a compound that partially or fully protects a crop plant of interest from the damage caused by a source of damage, preferably a herbicide, while leaving other plants with less efficient protection or preferably no protection at all. In a preferred embodiment, a plant other than said plant of interest is a weed species.

In a preferred embodiment, the term "pharmaceuticals" and animal health products, as described herein, refers to, but is not limited to, antibiotics, antiviral compounds, antiparasiticides

In a preferred embodiment, the term "antibiotic", as used herein, refers to a compound that detrimentally affects the growth and development and/or the ability to multiply and/or to infect of microbes, whereby said microbe is preferably selected from the group consisting of bacteria and eukaryotic microbes including fungi and protozoa. In a preferred embodiment, the antibiotic kills said microbe. In a preferred embodiment, the antibiotic can be used to treat humans, animals, plants and/or any other organism suffering from a microbial infection or to prevent them from getting or developing such infection.

In a preferred embodiment, the term "antiviral compound", as used herein, refers to a compound that detrimentally affects the ability of a virus to multiply and/or infect, its biological activity and/or its structural and/or chemical stability and integrity, respectively. In a preferred embodiment, the antiviral compound can be used to treat humans, animals, plants and/or any other organism suffering from a viral infection or to prevent them from getting or developing such infection.

In a preferred embodiment, the term "anti-parasite drug", as used herein, refers to a compound that detrimentally affects the growth, development, fertility and/or health of a parasite and/or its ability to multiply and/or infect. In a preferred embodiment, the anti-parasite drug kills said parasite. In a preferred embodiment, the anti-parasite drug can be used to treat humans, animals, plants and/or any other organism suffering from a parasitic infection or to prevent them from getting or developing such infection.

In a preferred embodiment, the term "nutrition-enhancing compound", as used herein, refers to a compound which adds to the benefit obtained from the consumption of any food used to feed humans, animals, plants and/or any other organism when added to such food. In a preferred embodiment, the term "nutraceutical", as used herein, refers to a compound added to food to elicit a medical effect when such food is fed to humans, animals, plants and/or any other organism.

The person skilled in the art is familiar with the use of libraries such as aptamer libraries, which contain up to 10¹⁵ or even more chemically different molecules (Ellington, A.D., supra, Methods in Enzymology, Vol 267, Combinatorial Chemistry (1996), Abelson J.N. and Simon M.I., eds., Academic Press, San Diego; Current Protocols in Nucleic Acid Chemistry (2007), last supplemented March 2007, Beaucage S.L., Bergstrom D. E., Herdewijn P., Matusda A., eds., John Wiley and Sons Inc. New York). The chemical nature of aptamers, in particular their ability to form a range of secondary structures, their high solubility and their sequence diversity makes them the molecules of choice for such applications. Using high-throughput screening methods it could be demonstrated that high-affinity aptamer ligands to protein targets can be isolated from aptamer libraries; such ligands often form extremely stable complexes (nanomolar dissociation constants) which, nevertheless, can still be resolved. The provision of such libraries can include the chemical synthesis and subsequent buffer exchange, but also the amplification of nucleic acid molecules using a template library or an enriched template library fraction as a template through suitable polymerases and/or reverse transcriptases. In any event, the library is in an aqueous solution, preferably a buffered solution.

In analogy, peptide aptamers which have been described to comprise a similar variety of secondary structures yielding libraries with comparable binding versatility as nucleic acid aptamers can be used as elements in the sense of this invention (M. Crawford, R. Woodman, P.K. Ferrigno (2003): Briefings in Functional Genomics and Proteomics Vol 2, pp 72-79 Peptide aptamers: Tools for biology and drug discovery; F. Hoppe-Seyler, I. Crnkovic-Mertens, E. Tomai, K. Butz (2004); Current Molecular Medicine, Vol 4, pp 529-538: Peptide Aptamers: Specific Inhibitors of protein function; I. C. Baines, P. Colas (2006): Drug Discovery Today, Vol 11, pp 334-341 Peptide Aptamers as guide for small molecule drug discovery).

In a further step, the library is mixed with the preparation comprising the interaction partner to be identified. This preparation could be any kind of fraction or extract derived from biological materials or fractions of biological materials that comprise the interaction partner. The person skilled in the art is aware of and trained to prepare such extracts, for example using detergents or blenders to prepare eukaryotic samples such as mammalian tissues or cell lines, using a freeze-thaw procedure to disrupt bacterial cells or freeze-drying and powdering plant materials (WO2001/056584; Current Protocols in Protein Science (2007), last supplemented May 2007, Coligan J. E., Dunn B.M., Speicher D.W., Wingfield P.T., eds., John Wiley and Sons Inc. New York, Current Protocols in Cell biology (2007), last supplemented March 2007, Bonifacino J.S., Dasso M. Harford J.B., Lippincott-Schwartz J., Yamada K.M. eds., John Wiley and Sons Inc. New York). Fractions of such extracts can be prepared according to procedures well known to the one skilled in the art, for example, preparative ultracentrifugation, enzymatic treatment (e.g. DNAses, RNAses or proteases) to degrade unwanted types of molecules, the addition of stabilisers, for example protease inhibitor cocktails, to prevent degradation of molecules of interest (Current Protocols in Cell biology (2007), last supplemented March 2007, Bonifacino J.S., Dasso M. Harford J.B., Lippincott-Schwartz J., Yamada K.M. eds., John Wiley and Sons Inc. New York). The preparation can also be made of other sources, for example environmental samples such as soil samples, which may comprise more than one organism. However, the preparation, in particular the solutions used, need to be compatible with physiological complex formation. Typically, a relevant preparation comprises an extracellular or cytosolic extract or a membrane fraction that contains a range of proteins and, hopefully, a protein that is the interaction partner of interest.

In a further step, an incubation is allowed under conditions that promote binding of the elements to the interaction partner. In such an incubation step measures may be taken by a person skilled in the art to increase the performance of such binding. These measures may include the addition of agents protecting the library elements. In case such elements are nucleic acids, the protecting agent is preferably an RNAse inhibitor or a DNAse inhibitor (Blackburn, P. and Moore, S. (1982) Pancreatic Ribonucleases. The Enzymes, XV, Part B, Academic Press, NY; Kim, B.M., Schultz, L.W. and Raines, R.T. (1999). Protein Sci. 8, 430-4; Junowicz, E. and Spencer, J. (1973), Biochim Biophys Acta 312, 85). It is anticipated that, given the enormous chemical diversity of the molecules in the library, a multitude of binding sites, among them the binding site of the active agent on the interaction partner, are then involved in complex formation and some of which will be saturated with binding elements at the end of the incubation period. In a most preferred embodiment, the library is sufficiently diverse and the candidate interaction partners are sufficiently stable to allow for saturation of all possible binding sites with elements.

In a further step, non-binding elements are removed from the solution. The particular technique to be used depends on the nature of the library, the conditions and the preparation comprising the interaction partner. The person skilled in the art is familiar with a range of suitable techniques applicable under such conditions. Preferably, if the library comprises aptamers and the preparation comprising the interaction partner includes proteins, the non-binding elements can be removed by filter retention, pull down assays, preparative ultracentrifugation, size exclusion chromatography, affinity chromatography, native gel electrophoresis or the like (Joyce G.F. Curr Opin Struct Biol. 1994;4:331-6.; Phage Display of Peptides and Proteins: A Laboratory Manual (1996), Kay B.K., Winter J., McCafferty J. eds., Academic Press, San Diego; Methods in Enzymology, Vol 267, Combinatorial Chemistry, (1996), Abelson J.N. and Simon M.I., eds. Academic Press, San Diego; Current Protocols in Nucleic Acid Chemistry, 2001-2007, last supplemented March 2007, Beaucage S.L., Bergstrom D. E., Herdewijn P., Matusda A., eds., John Wiley and Sons Inc. New York). Finally, after a final washing step that can be included in a preferred embodiment of the invention, only molecules stemming from the preparation comprising the interaction partner should be present, some of them in complex with elements, and many, ideally all of their binding sites should be saturated with elements.

In a further step, elements that bind to the binding site of the active agent on the interaction partner are released. Typically, an excess of the active agent is added to the effect that elements binding to said binding site are displaced or released from the complex by means of elution. Depending on the relative stabilities of the complexes formed between the interaction partner on the one hand and the binding element and the active agent, respectively, on the other hand, suitable reaction conditions, in particular with respect to time and the concentration of the active agent, can be determined using routine procedures well known to the person skilled in the art. At the end of this step, all elements that were not released from the interaction partner, by competitive or non-competitive mechanisms, should still be bound to candidate interaction partners except for those previously bound to the interaction partner, as the latter have just been displaced by the active agents.

In a further step, the mixture comprising uncomplexed candidate interaction partners, candidate interaction partners in complex with elements, free active agent molecules, the interaction partner in complex with the active agent, and released elements may be partitioned for the purpose of isolating the binding element. In a more preferred embodiment, if the interaction partner is part of a proteome, the active agent is a small molecule and the element is an aptamer, the use of techniques similar to those discussed in the context of removing the non-binding element (e.g. filter retention assays, preparative ultracentrifugation, size exclusion chromatography, affinity chromatography or native gel electrophoresis) ((Joyce G.F. Curr Opin Struct Biol. 1994;4:331-6.; Phage Display of Peptides and Proteins: A Laboratory Manual (1996), Kay B.K., Winter J., McCafferty J. eds., Academic Press, San Diego; Methods in Enzymology, Vol 267, Combinatorial Chemistry, (1996), Abelson J.N. and Simon M.I., eds. Academic Press, San Diego; Current Protocols in Nucleic Acid Chemistry, 2001-2007, last supplemented March 2007, Beaucage S.L., Bergstrom D. E., Herdewijn P., Matusda A., eds., John Wiley and Sons Inc. New York), is adequate. Optionally, the isolated elements can, subject to the elements being nucleic acids, be amplified provided this is technically feasible, for example through the use of suitable polymerases/reverse transcriptases if the element is a nucleic acid that can be enzymatically amplified by means of such polymerase/reverse transcriptase. Preferably, the constant moiety of elements such as nucleic acids is designed for such purposes. Moreover, one or several of the preceding steps of the method of the present invention can optionally be repeated once or more often, typically and preferably resulting in one or a number of consecutive enrichment cycles ("cycling") under the proviso that all of the steps required to perform such cycle, are actually performed, for a more stringent selection of an element and more preferably of a nucleic acid that binds to the binding site of the active agent on the interaction partner.

Without wishing to be bound to any theory, the present inventors assume that the binding element can be assumed to bind specifically to the binding site of the active agent on the interaction partner given that it has been released through competitive or non-competitive binding of the active agent. It is, however, also within the present invention that the binding element is released through a mechanism which is different from a competitive binding mechanism. Insofar, for example, also an allosteric mechanism is encompassed in an embodiment of the present invention. In such a case, the binding of the active agent to a site at the interaction partner which is different from the binding site of the binding element will result in the release of the binding element from the interaction partner. In an embodiment of the invention, a binding element will be released from the interaction partner(s) as a result of an interaction of the interaction partner(s) with the active agent(s), indicating that the interaction partner(s) of the active ingredient and the eluted element(s) are preferably either identical or functionally linked to such an extent that binding of the active agent will result in the release of the binding element(s). Preferably, though, the element is chemically versatile and can be used to isolate the interaction partner in subsequent steps. Typically, the element is an aptamer, which can be immobilised on a support. There are a number of techniques well known to the person skilled in the art that can be used to immobilise an element, for example biotinylation followed by immobilisation using streptavidin beads, ion exchange chromatography (immobilisation *via* the negatively charged backbone of the nucleic acid molecule and positive charges on the support) or the use of immobilised nucleic acid capture probes partially complementary to the binding nucleic acid molecule. In a preferred embodiment, the immobilisation of the binding element consisting of a constant moiety and a variable moiety, whereby the constant moiety is identical to or in a group of or all of the elements of the element library, and the variable moiety is different within a group of or among all of the elements of the element library, is mediated by the constant moiety of the element. However, it is also within the present invention any part or moiety of the element can be used for such purpose. There are equivalent or different methods if the element is a peptide, for example biotinylation, the use of immobilised antibodies, coupling *via* cystein and lysine residues, and by affinity tags such as His-Tag GST and Strep-Tag which are known to a person skilled in the art. Methods for the immobilisation of molecules are, e.g. described in Hermanson G.T. (1996), Bioconjugate Techniques, Academic Press, San Diego, CA 92101-4495, USA, or in Blank, M. et al., J Biol Chem. 2001; 276(19):16464-8, Current Protocols in Protein Science, 2007, last supplemented May 2007, Coligan J. E., Dunn B.M., Speicher D.W., Wingfield P.T., eds., John Wiley and Sons Inc. New York and Morris K.N., Proc. Nat. Acad. Sci 1998, 95:2902-2907. However, the immobilisation step is entirely optional.

In another step, the binding element, which, in an embodiment of the invention, can be amplified prior to setting up this step, is placed in contact with a preparation comprising the interaction partner, preferably the same preparation and under the same conditions as used to contact the library, allowing, again, for formation of the complex between the interaction partner and the binding element. Typically, the complex is formed between an immobilised aptamer which acts as an affinity ligand to capture the protein interaction partner in the preparation comprising the interaction partner. However, the immobilisation step at this point is not mandatory; alternatively a complex can be formed in solution and can subsequently be isolated, for example using a biotinylated aptamer that is exposed to streptavidin beads following complex formation. In this context, it should be pointed out that some moieties of the element, preferably constant moieties, can be assumed not be involved in the binding event and can be used for a chemical modification required for the isolation of the complex or the immobilisation of the binding element. Should the chemical modification be a point of concern with respect to the binding properties of the binding element according to the present invention, the library can be modified (*e*.*g*. biotinylated) prior to exposure to the preparation comprising the interaction partner (the first exposure, step b) of the method according to the present invention), ensuring that elements selected according to the present invention bind to the interaction partner nonwithstanding the fact that they carry a chemical modification.

In a further step, the interaction partner is characterised. A wide range of methods suitable for the characterisation of the interaction partner are available and well known to the person skilled in the art. Typically, the complex consists of an immobilised aptamer that binds to a proteinatious interaction partner. In such a case, the complex may be washed, and the interaction partner and, in a preferred embodiment, may be eluted using the active agent. The preferably isolated interaction partner can then be subjected to a variety of analytical techniques such as proteolytic digest and/or analysis by mass spectrometry, MALDI-MS, MALDI-TOF-MS, sequencing techniques or functional tests including binding assays, activity assays and immunological methods (*e*.*g*. Western blot) for the purpose of characterisation (Mann M. et al. (1993), Biol Mass Spectrom 22:338-345; Current Protocols in Protein Science (2007), last supplemented May 2007, Coligan J. E., Dunn B.M., Speicher D.W., Wingfield P.T., eds., John Wiley and Sons Inc. New York Sambrook J., Russel D.W. (2001), supra; Current Protocols in Nucleic Acid Chemistry (2007), last supplemented March 2007, Beaucage S.L., Bergstrom D. E., Herdewijn P., Matusda A., eds., John Wiley and Sons Inc. New York; Dixon M. and Webb E.C., Enzymes 2nd ed. (5th impression), Longmans, Green and Co LTD, London).

Alternatively, if the interaction partner is a protein in complex with an element (*e*.*g*. a biotinylated aptamer) and in solution, the complex can be purified (*e*.*g*. using streptavidin beads). A number of methods are well known to the person in the art to separate such a complex, *e*.*g*. through the use of gel electrophoresis, and the same suite of techniques as mentioned above can then be used to characterise the interaction partner.

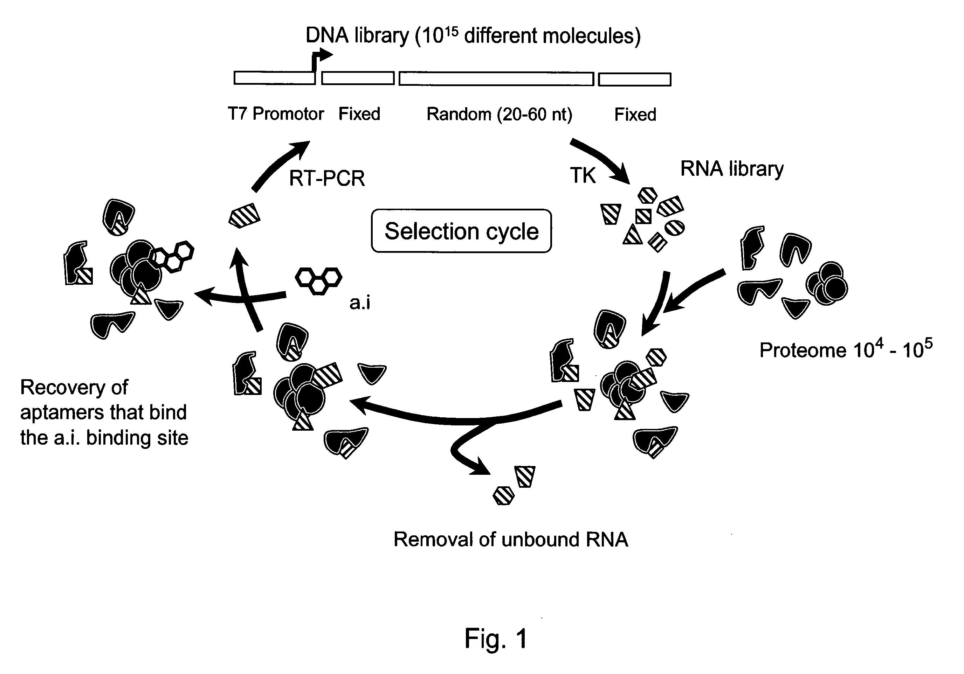

The present invention is further illustrated by the following figures and examples from which further features, embodiments, aspects and advantages of the present invention may be taken.
More specifically, Fig. 1 schematically depicts the various steps of an embodiment of the method - the SELEX process - whereby TK means RNA transcription; RT-PCR means reverse transcription-polymerase chain reaction;

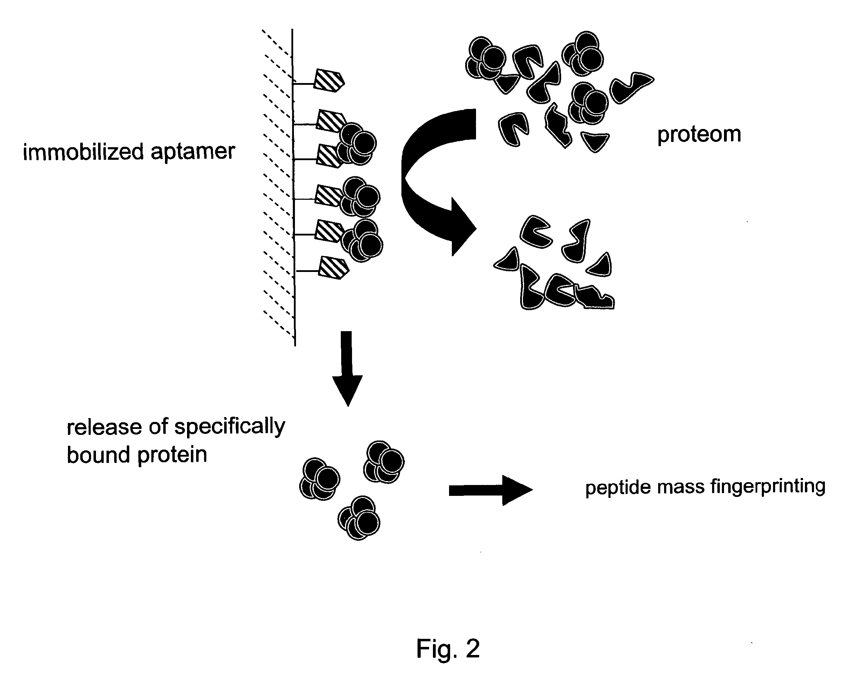

Fig. 2 schematically depicts one particular step of the method according to the present invent where RNA molecules, which have been identified as specifically binding to the interaction partner and which can be displaced therefore by the active agent are immobilised to purify the interaction partner.

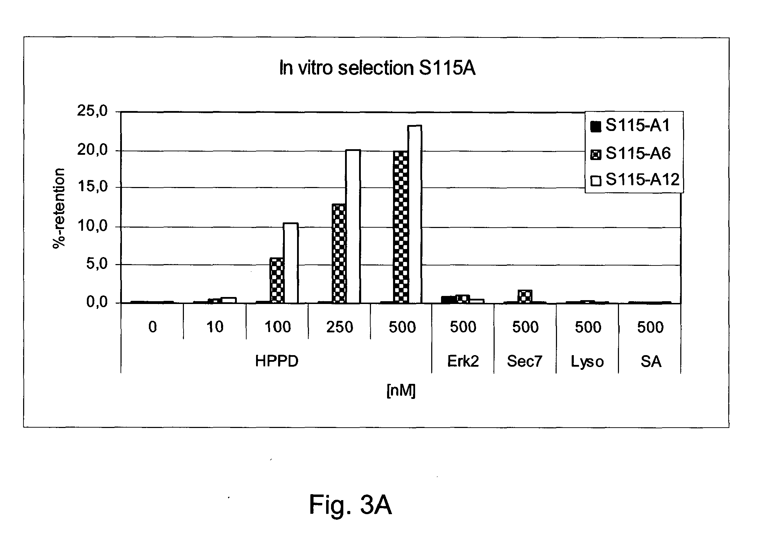

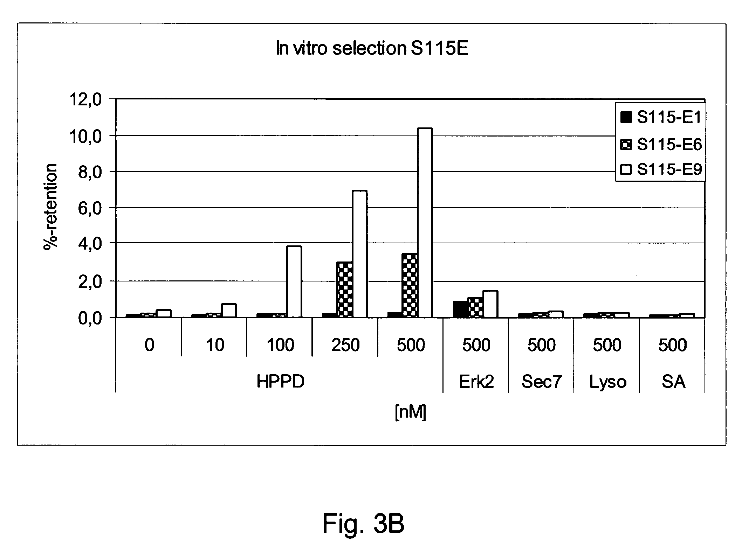

Fig. 3A and 3B show the results of a filter retention assay using aptamer libraries enriched in a classical SELEX experiment aimed to generate aptamers binding to HPPD, with the number of selection cycles being 12 (3A) or 9 (3B), and various concetrations of HPPD as well as control proteins;

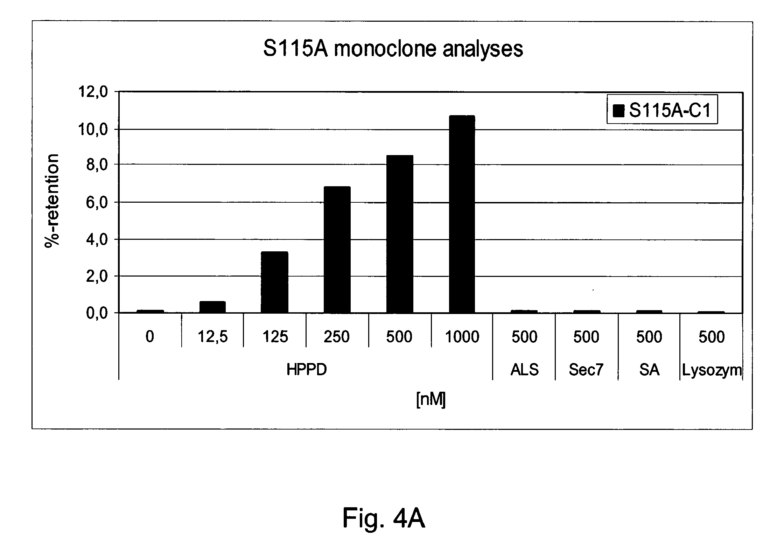

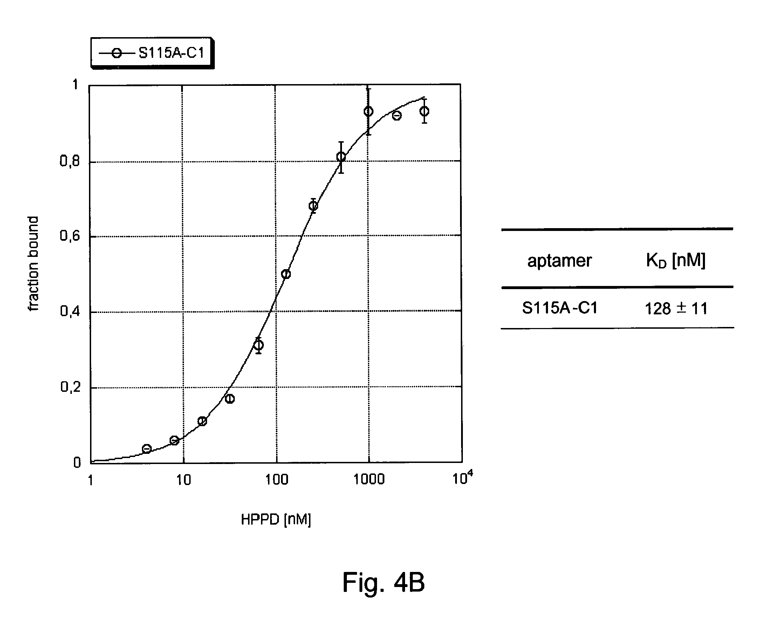

Fig. 4A shows a diagram depicting the results of a filter retention assay using a monoclonal aptamer (S115A-C1) generated in the previous experiment as the binding RNA molecule and HPPD, at various concentrations, as the interaction partner. Fig. 4B shows the analysis of such data for the purpose of calculation the dissociation constant;

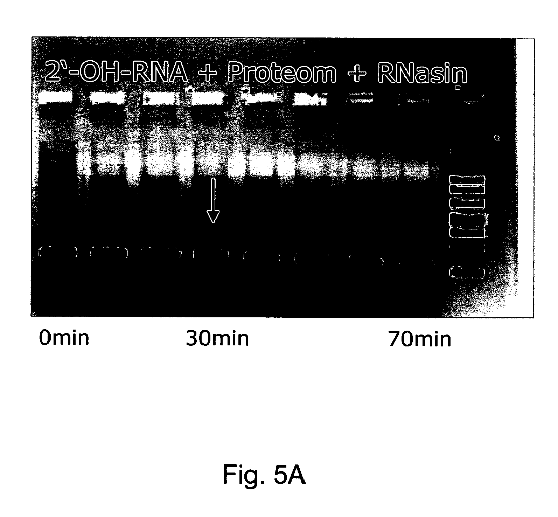

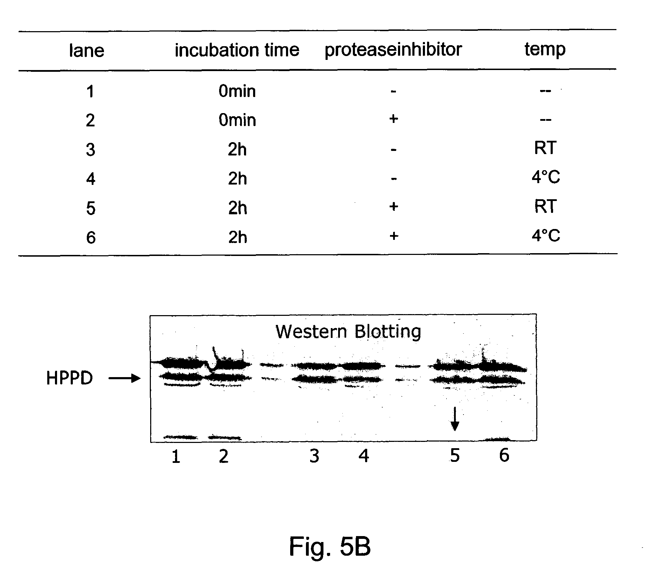

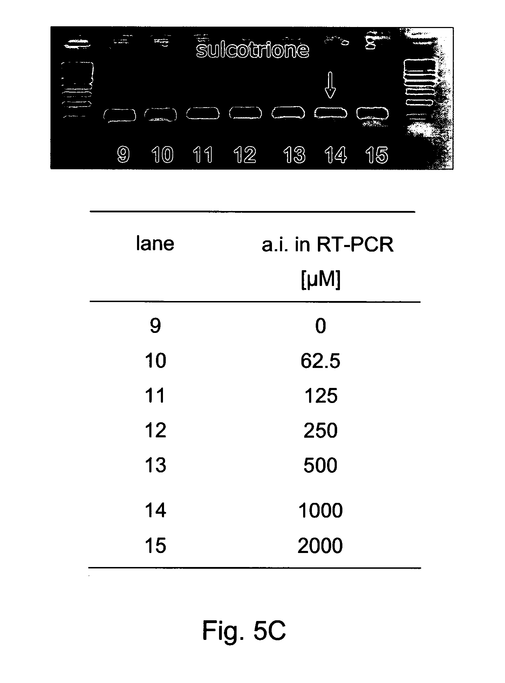

Fig. 5A shows an agarose gel electrophoresis run aimed to analyse the stability of a SELEX RNA molecule pool in the presence of protein extract and RNAse inhibitor (RNasin). Fig. 5B is a Western Blot showing the stability of recombinant, His-tagged HPPD in the presence of protein extract under various conditions. Fig. 5C shows an agarose gel electrophoresis run depicting the effect of various concentrations of the active agent (a.i., sulcotrione) on the amplification of RNA molecules through RT-PCR);

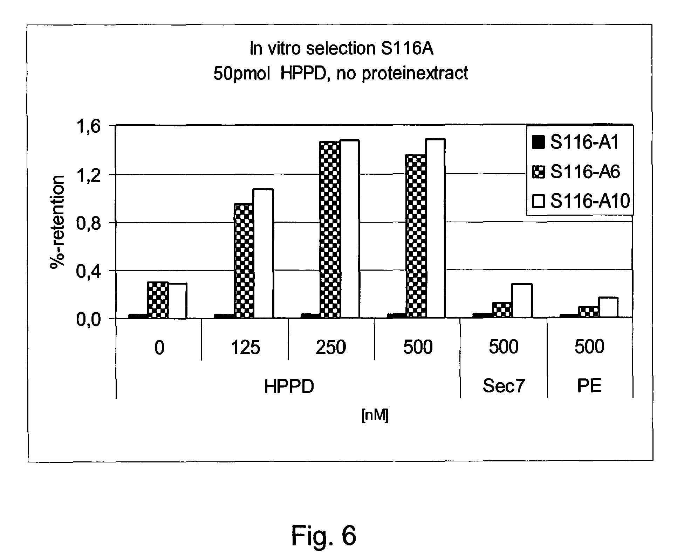

Figs. 6A depicts a diagram showing the results of filter retention assays using RNA molecules generated by a competitive SELEX protocol using recombinant HPPD as the interaction partner and sulcotrione as active agent;

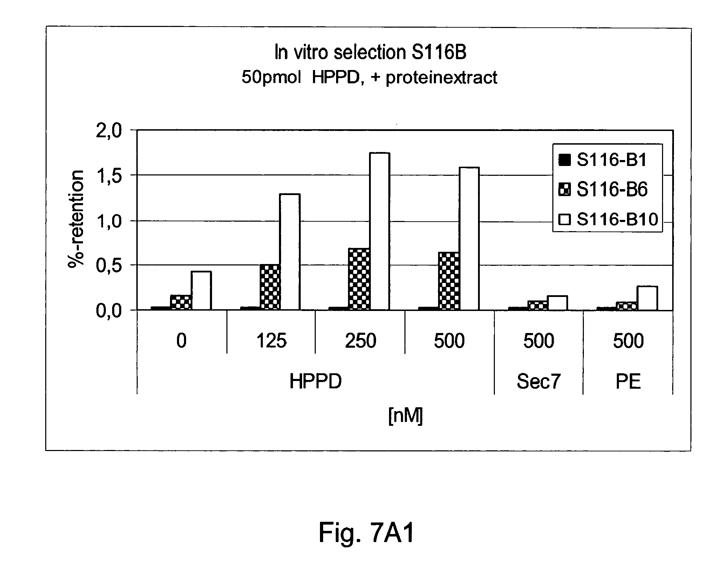

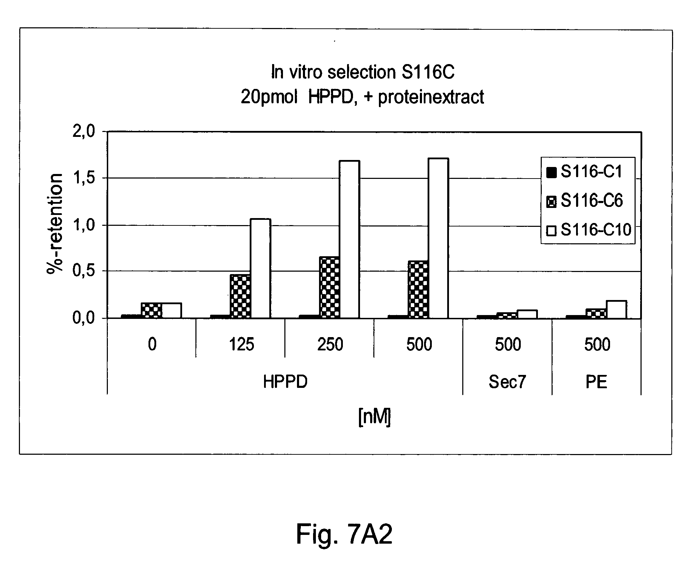

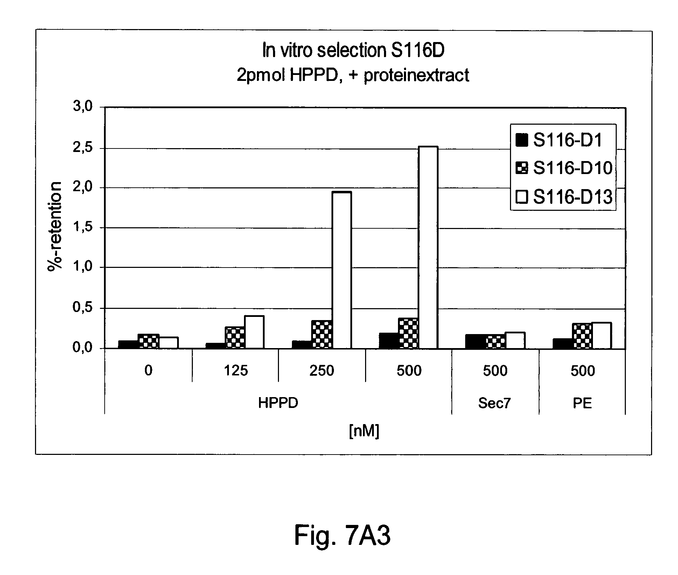

Figs. 7A1, 7A2 and 7A3 depict diagrams showing the results of filter retention assays performed to assess the binding properties of enriched aptamer libraries isolated in competitive SELEX runs using 50 pmol of HPPD (Fig. 7A1), 20 pmol of HPPD (Fig. 7A2) and 2 pmol of HPPD (Fig. 7A3) as the interaction partner in the presence of protein extracts during the selection cycles. As for the retention assays, the binding of the enriched aptamer libraries to various concentrations of the recombinant interaction partner as well as control proteins was assessed;

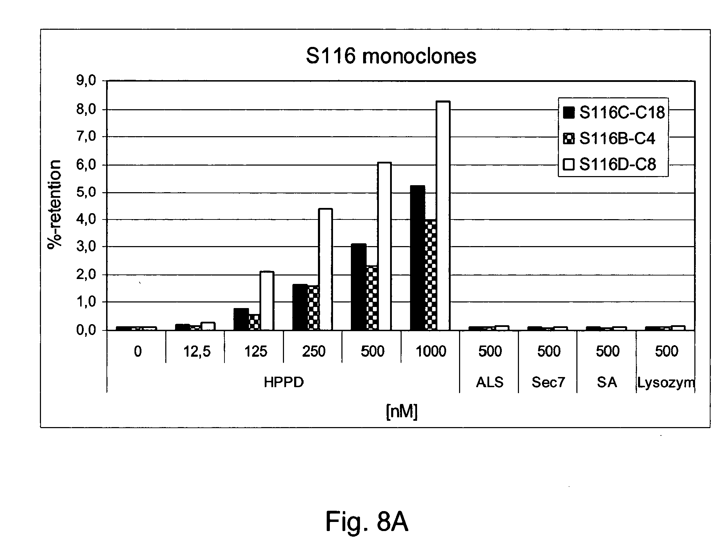

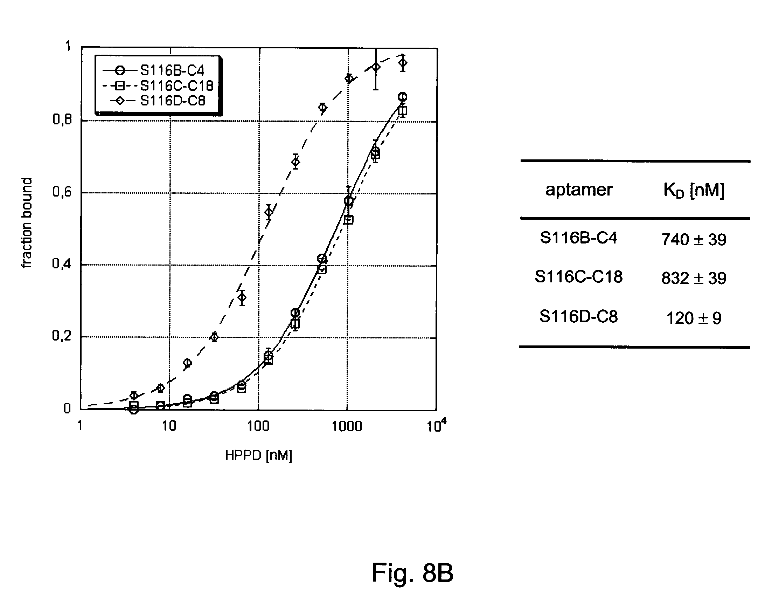

Figs. 8A and 8B depict diagrams showing the results of two filter retention assays (and the analysis of such data aimed to characterise the binding properties of three aptamer monoclones binding to HPPD, isolated in a competitive SELEX run;

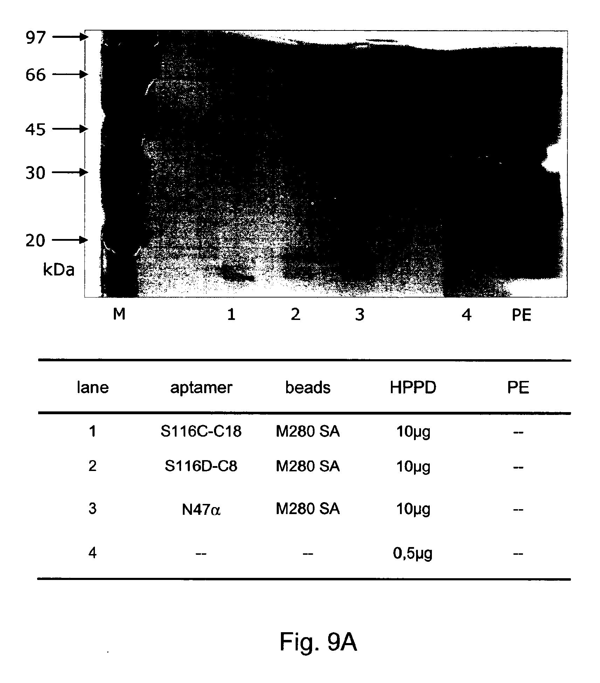

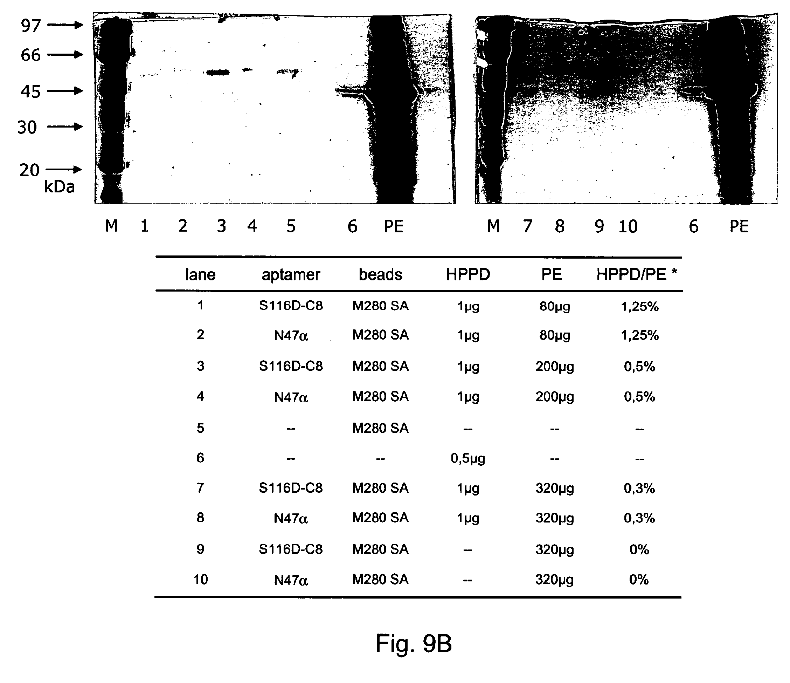

Figs. 9A and 9B depict SDS-PAGE gels showing the results of pull down experiments using monoclonal anti-HPPD aptamers immobilised on streptavidin beads as a capture probe to bind recombinant HPPD (9A, lanes 1 and 2) or endogenous HPPD (9B, lane 9) under various conditions; unselected RNA library (N47α) was used as negative control; HPPD migrates at approximately 45 kDa (compare 9A, lane 4 and 9B, lane 6, HPPD positive control);

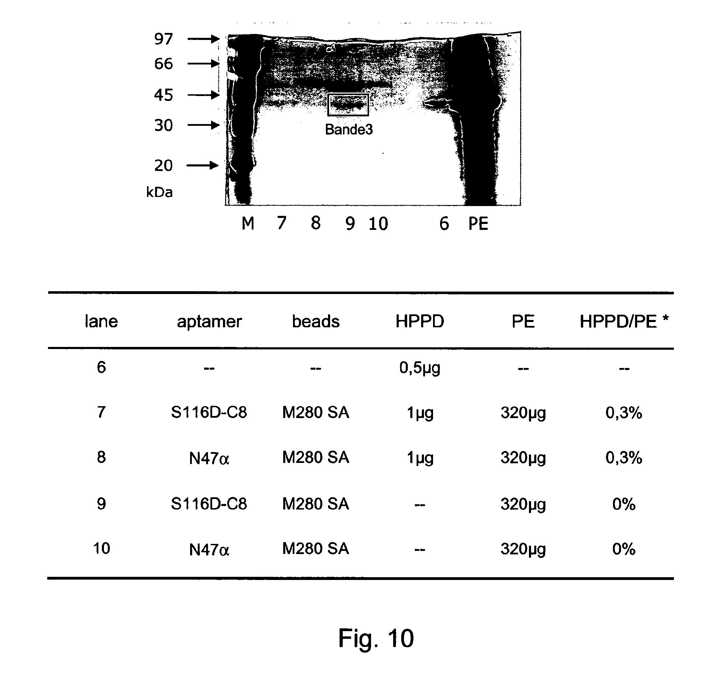

Fig. 10 depicts an SDS-PAGE gel showing the results of a pull down assay using monoclonal anti-HPPD aptamers immobilised on streptavidin beads as a capture probe to identify HPPD as the interaction partner through isolation of recombinant HPPD spiked into protein extract or endogenous HPPD (lane 9, "Bande 3") from protein extract.

### EXAMPLES

### Example 1: Schematic representation of the method according to the present invention

Figs. 1 and 2 illustrate an embodiment of the method according to the present invention for the identification of an interaction partner to an active agent. Firstly, a DNA library is provided that includes approximately 10¹⁵ molecules, each of them comprising a T7 promoter, a fixed sequence element, a random sequence element (20-60 nucleotides) and another fixed sequence element. This DNA library is transcribed (TK) to generate an RNA library. The RNA library is contacted with a proteome comprising approximately 10⁴-10⁵ molecules, among them the interaction partner of interest. After formation of a complex between the interaction partner and a binding RNA molecule, unbound RNA molecules are removed. Next, the proteome comprising proteins in complex with RNA molecules, among them the interaction partner in complex with a binding RNA molecule, is contacted with the active agent, leading to the release of the binding RNA molecule. The latter can be subjected to an amplification by reverse transcription and PCR, which allows either to clone the released RNA molecules into a cloning vector for sequencing or to amplify the binding RNA molecule (which is in fact typically a mixture of several binding RNA molecules) to generate an enriched library, which can subsequently be used for at least one more selection cycle which preferably aim for higher binding specificity.

As illustrated in Fig. 2, the binding RNA molecules immobilised on a support can subsequently be used to purify the interaction partner from a preparation comprising the proteome, preferably the same preparation as used in the selection cycle, *via* affinity chromatography. The purified interaction partner is subjected to peptide mass fingerprinting in order to establish its identity.

### Example 2: Accessibility of model targets to SELEX based-approaches

As a proof of principle, the method of the present invention was applied to the *Arabidopsis thaliana* system and one active agent, sulcotrione, which inhibits the known *Arabidopsis thaliana* interaction partner p-hydroxyphenylpyruvate dioxygenase (HPPD) (Schulz, A., Ort, O., Beyer, B., and Kleinig, H. (1993) FEBS Lett. 318 (2), 162-166; Secor, J. (1994) Plant Physiol. 106, 1429-1433), respectively. It was attempted to provide a binding nucleic acid molecule selected from a library comprising ribonucleic acid molecules amplified from a DNA template library. If this can be done, and if such ligands can be used to identify the two interaction partners, the feasibility of the above mentioned approach is verified.

In order for the method of the invention to be applicable, the interaction partner needs to be accessible to SELEX (Systematic Evolution of Ligands by Exponential enrichment)-based approaches. The suitability of interaction partners, in particular proteins, with respect to SELEX differs significantly. While it is straightforward to separate high-affinity nucleic acid molecules to some proteins using SELEX based methods, the yield of high-affinity ligands with respect to other proteins may be reduced.

The accessibility of HPPD to SELEX-based approaches was evaluated in a set of pilot experiments comprising a classical SELEX-type of experiment.

Recombinant, His-tagged HPPD was targeted as an interaction partner in two SELEX runs, referred to as S115A and S115E, which comprised 12 and 9 cycles, respectively. Briefly, the protein was biotinylated and exposed to the RNA library with a 47 nucleotide random region (N47α) in 20 mM Hepes (pH 7.4), 150 mM NaCl, 3 mM MgCl₂, and 1 mg/ml BSA (in vitro selection S115A) or 20 mM Hepes (pH 7.4), 150 mM NaCl, 3 mM MgCl₂, 10 % DMSO and 1 mg/ml BSA (in vitro selection S115B). The library N47α consists of a random region of 47 nucleotides which is flanked by fixed sequences at the 5' and 3' end that serve as primer annealing sites for the amplification of the selected aptamer sequences via reverse transcription and PCR. The 5' region additionally contains the sequence of an T7-RNA polymerase promoter that enables the transcription of the PCR DNA template into RNA. The sense sequence of the PCR DNA template is "5'-AATTCTAATACGACTCACTATAGGGAGAGGAGGGAGATAGATATCAA-N47-AGTTTGTCCTCACGGTGGATGG-3' (SEQ. ID No. 1). The T7-RNA polymerase promoter is underlined. The double stranded DNA template was used to transcribe the initial RNA library. The procedures to produce an RNA library and to perform a SELEX experiment are well known to a person skilled in the art and described in, e.g., Methods in Enzymology, Vol 267, Combinatorial Chemistry (1996), Abelson J.N. and Simon M.I., eds. Academic Press, San Diego. Subsequently, the complexes between target and RNA were captured on streptavidin-coated beads and unbound RNA was removed by washing with binding buffer. The bound RNA molecules were eluted by heating to 95°C and amplified by RT-PCR and in vitro transcription before starting the next selection cycle. A nitrocellulose filter retention assay was used to determine binding of selected, radioactively labelled RNA libraries to recombinant protein (Methods in Enzymology, Vol 267, Combinatorial Chemistry, (1996), Abelson J.N. and Simon M.I., eds. Academic Press, San Diego). RNA library (∼80nM) was incubated with increasing concentrations of target protein in binding buffer and subsequently filtered through nitrocellulose filters which bind to proteins but not to RNA molecules. RNA bound to the target protein is retained within the complex on the filter and was quantified by a phosphoimager. Values were calculated as the percentage of total radiolabeled RNA in the reaction. Fig. 3A and B show that up to approximately 20-25% (in vitro selection S115A) and approximately 10% (in vitro selection S115B) of the labelled RNA libraries are retained in the presence of 500 nM HPPD, while virtually no binding could be detected if HPPD was replaced by an equivalent concentration of control proteins as negative controls. The experiment S115B also demonstrates that enrichment of binding activity was successful in the presence of DMSO which can be important to dissolve small molecule active agents.

To evaluate the binding affinity of selected aptamers, the library enriched with respect to aptamers binding to HPPD was cloned after cycles 6 and 12 (S115A) and cycle 9 (S115E). 60 clones were sequences, and 11 of them could be shown to bind to HPPD.

This is exemplified by aptamer S115A-C1 (sequence: 5'-GGGAGAGGAGGGAGAUAGAUAUCAACAAUGACUAGCCGUGUAAGUGGUCUC GUACAUCCGAAAAAGACGACCCAGUUUGUCCUCACGGUGGAUGG-3') (SEQ. ID. No. 2), which was retained in the above mentioned assay (Fig. 4A) by HPPD, but not by any of the control proteins. The dissociation constant (Fig. 4B) was determined to be 128 ± 11 nM, consistent with strong binding to S115A-C1 to recombinant HPPD.

Therefore, HPPD appears to be accessible to classical SELEX suggesting that it may be possible to identify HPPD as an interaction partner of sulcotrione using the method according to the present invention based on a competitive SELEX approach.

### Example 3: Establishing suitable reaction conditions for complex formation

After the preliminary SELEX experiment, pilot experiments were carried out to establish suitable conditions to test the method according to the present invention.

When a protocol for the formation of aptamer-protein complexes is to be devised, it should be borne in mind that, in the absence of any information on the binding kinetics, a sufficiently long period of time must be allowed for formation of complexes with low kₒₙ values. On the other hand, both endogenous proteins and chemically synthesised oligonucleotides are subject to cellular degradation processes, and the binding activities of proteins may decrease over time, so researchers aim to minimise the contacting time. Therefore, it was attempted to establish suitable conditions for the complex formation step.

In a first experiment, His-tagged recombinant proteins HPPD and RNA library N47α were incubated with protein extract under varying conditions. The integrity was subsequently analysed.

First, protein extract was prepared from freeze-dried powder of *Arabidopsis thaliana* according to the following protocol. All steps were carried out on ice. 500 mg freeze-dried powder were suspended in 500µl ice-cold buffer (PBS, 3mM MgCl₂+1/100 HALT protease inhibitor cocktail (Pierce)) and homogenized with a hand-held homogenizer on ice for one minute. Addition of 200µl ice-cold buffer was followed by brief vortexing. The slurry was centrifuged in two 1.5 ml Eppendof-cups (15 min, 18,000 x g, 4°C), the supernatant saved, the pellets were re-suspended in additional 400 µl buffer each and once again homogenized for 30 sec on ice. The slurry was centrifuged (15 min, 18,000 x g, 4°C) and all supernatants were collected. The concentration of the protein extract was determined using the BCA assay kit of Pierce (Adilakshami, T. and Laine, R.O. (2002), J. Biol. Chem. 277:4147-51. Fischer, T., et al. (1999) Proc. Nat. Acad. Sci. 96:6722-7).

The stability of the RNA library was assayed by incubating RNA molecules (200 pmol in 200 µl of protein extract supplemented with RNAse inhibitors (RNasin 0,4 U/µl, Promega) at 4°C) and subjecting aliquots removed from the sample after various time points to agarose gel electrophoresis (Fig. 5A). Only moderate degradation was detected over 70 min enabeling an optimal incubation of the RNA library with the protein extract for 30 min in the subsequent in vitro selections. Same experiment was repeated at room temperature with similar outcome.

Next, the stability of the target proteins in the presence of protein extract (100µl supplemented with 2.5 µl HALT protease inhibitor cocktail (commercially available from Pierce) were indicated) was evaluated by Western blotting using recombinant His-tagged HPPD (final concentration: 0.1 µg/µl) and a commercially available anti-His antibody (Penta-HIS-Alexa Fluor 488 Conjugat, Qiagen) and secondary antibody anti-mouse IgG AP-conjugat (Promega)). The target level was assessed after an incubation period of two hours under four different conditions (in the absence/presence of HALT Protease Inhibitor Coctail, EDTA-free (commercially available from Pierce) at room temperature/4 °C). In all cases, significant amounts of HPPD could be detected (Fig. 5B). Therefore, both proteins are reasonably stable in protein extract even in the absence of protease inhibitor cocktail at room temperature for the duration of the incubation period suggested by the previous experiment.

### Example 4: Excluding interference of the active ingredient with the amplification steps

Upon release of the binding aptamer through displacement, both, the binding aptamer and a high concentration of the active agent are present. It is a prerequisite for the efficient amplification of the aptamer, a step that is essential if several cycles of SELEX are to be performed, that the active agent does not interfere with the amplification steps (i.e. RT-PCR) carried out to increase to amount of enriched aptamers. In a typical experiment, 50µl elution solution will be added to 50 µl RT-PCR mix which means that the concentration of the active agent in the RT-PCR reaction will be as high as 50% of the elution. Therefore, a control experiment was performed to evaluate whether the concentrations of active agents required for the competitive elution of the binding aptamers interfere with the amplification step. Fig. 5C shows that a significant amount of amplified DNA after RT-PCR of N47α RNA library as template (One step RT-PCR kit commercially available from Qiagen) can be obtained at concentrations as high as 2000 µM (2 mM) of sulcotrione, demonstrating that this active agent does not impair the efficiency of the amplification steps.

From these results it is obvious that HPPD is accessible to SELEX-based approaches, and suitable reaction conditions for complex formation and the amplification could be established, encouraging the application of the method according to the present invention to the HPPD/sulcotrione system of interaction partners and active agents.

### Example 5: Evaluating whether binding aptamers can be enriched through SELEX in combination with competitive elution using purified interaction partners

Next, experiments were carried out to confirm that aptamers binding to HPPD can be enriched through SELEX and can subsequently be eluted using the established concentrations of sulcotrione, the active agent. A competitive RNA selection protocol similar to the one used in Example 2 was followed using 50 pmol of HPPD as interaction partner, but binding aptamers were eluted by addition of sulcotrione to a final concentration of 2 mM instead of heating to 95 °C. A total number of 10 and 16 SELEX cycles were run in the case of HPPD (experiment S116A).

Enriched RNA libraries were subjected to the same filter retention assay as used in Example 2. Fig. 6A shows that a significant amount of radiolabelled RNA could be captured in a saturable manner using HPPD after 6 or 10 SELEX cycles, while virtually no RNA interacted with control protein Sec7 and with protein extract, showing that aptamers binding specifically to HPPD could be isolated using the competitive SELEX approach.

### Example 6: Evaluating whether binding aptamers can be enriched through SELEX in combination with competitive elution under near-physiological conditions

While the results of Example 5 provide strong support for the strategy underlying the method according to the present invention, another set of experiments with a similar set up was performed; however, protein extract (8µg protein/100µl) and decreasing concentrations of purified interaction partners were used to approach physiological conditions with respect to complex formation. A significant amount of aptamers were retained in the experiments using a combination of purified HPPD and protein extract (Fig. 7A1, 7A2 and 7A3). If 50 pmol of HPPD were used (experiment S116B), six SELEX cycles were sufficient to obtain binding aptamers, although the yield could be dramatically increased if the number of cycles were elevated to 10. Moreover, the binding aptamers among the generated mixture of aptamers appeared to be reasonably saturated with interaction partner molecules at a concentration of 250 nM of HPPD, strongly suggesting a nanomolar dissociation constant with respect to complex formation. There was virtually no binding to the control protein Sec7 or protein extract which confirms that the binding was highly specific. To determine the amount of interaction partner required, the experiment was repeated using lower amounts of HPPD. Decreasing the amount of HPPD from 50 pmol to 20 pmol (experiment S116C) did not influence the outcome of the experiment significantly. If the amount of HPPD was decreased to 2 pmol (experiment S116D) the amount of binding aptamers retained in the filter retention assay only slightly exceeded the amount retained by protein extract or the control protein; however, this could be compensated by increasing the number of SELEX cycles to 13, reducing the proportional level of the binding aptamers with respect to HPPD and the negative control, respectively, to the level observed in the two previous experiments.

Overall, the data presented in this example demonstrate that the method according to the present invention could be used with great success to isolate a mixture of aptamers that comprise likely high affinity ligands to HPPD, even if near-physiological conditions (i.e. low amounts of HPPD and the presence of protein extract comprising multiple candidate interaction partners) were applied.

### Example 7: Characterising the binding properties of aptamers isolated as high affinity ligands to HPPD

To determine the binding properties of individual aptamers isolated as part of the mixture of aptamers binding to HPPD in the previous example, aptamers were cloned into a vector, and 24 clones were sequenced. For each experiment out of S116B, S116C and S116D, one of the cloned aptamers subsequently identified as specific ligands to HPPD (termed S116C-C18 (5'-GGGAGAGGAGGGAGAUAGAUAUCAAUAUCGCCACGUUACGAGUACCAAGCUU UAUUUCCGCAGCCCGAUAGAGUUUGUCCUCACGGUGGAUGG-3' (SEQ. ID. No. 3)), S116B-C4 (5'-GGGAGAGGAGGGAGAUAGAUAUCAAGUAACAAAUGAUA CUUUCCAAGCAUAAUUCAUUCGCAGCCCGAAUUAAGUUUGUCCUCACGGUGG A UGG-3' (SEQ. ID. No. 4)) and S116D-C8 (5'-GGGAGAGGAGGG AGAUAGAUAUCAAUAAAGUCGGUGACACCACGCAUCCA AAUACCGGUACGCGGAACUAAUAGUUUGUCCUCACGGUGGAUGG-3') (SEQ. ID. No. 5)) was analysed with respect to the thermodynamics of the binding. The three monoclonal aptamers were subjected to the nitrocellulose filter retention assay discussed in Example 2 (Fig. 8A and 8B). The dissociation constants determined for S116B-C4, S116C-C18 and S116D-C8, were 740 ± 39, 832 ± 39 and 120 ± 9 nM, respectively, consistent with the view that decreasing the concentration of the interaction partner to 2 pM (experiment S116D) actually yield the most tightly binding aptamer.

### Example 8: Using tightly-binding, immobilised aptamers to pull down recombinant interaction partner

To confirm the concept that aptamers isolated using the competitive SELEX approach can be used to identify the interaction partner of interest, the two tightly binding aptamers S116C-C18 and S116D-C8, henceforth referred to as anti-HPPD aptamers, were biotinylated by in vitro transcription, using the appropriate DNA template and biotinylated ApG starter nucleotides (IBA, Göttingen) (Pitulle C. et al. (1992) ,Gene, 112(1):101-5; Gaur R.K. et al. (2004), Methods Mol Biol. 252:9-17) and immobilised on paramagnetic streptavidin (SA) beads. Immobilised aptamers were then exposed to 10 µg of HPPD in incubation buffer (20 mM Hepes (pH 7.4), 150 mM NaCl, 3 mM MgCl₂, 10 % DMSO and 10 µM tRNA) in the absence of *Arabidopsis thaliana* protein extract (PE) and washed four times using washing buffer (20 mM Hepes (pH 7.4), 150 mM NaCl, 3 mM MgCl₂, 10 % DMSO and 1 µM tRNA. Aliquots of samples including either one of the two aptamers or N47α RNA library as control coupled to beads were analysed by sodium dodecyl sulphate Polyacrylamid gel electrophoresis (SDS-PAGE) (Fig. 9A). Indeed, both binding aptamers could be used to pull down HPPD as judged by the presence of a protein band that runs at the same height as a sample of purified HPPD (and, important with respect to later experiments, slightly higher than the main band in PE), while this band was absent when beads only were used to attempt to pull down HPPD.

This result shows that it is straightforward to chemically modify the two aptamers selected through the competitive SELEX approach according to the present invention for the purpose of immobilisation without compromising their ability to bind to HPPD.

After a range of pilot experiments suggesting the use of 20 mM Hepes (pH 7.4), 150 mM NaCl, 3 mM MgCl₂, 10 % DMSO, 0,05 % Tween as washing buffer were carried out to determine suitable assay conditions, aptamer S116C-C8 was used for pull down assays using recombinant HPPD in the presence of increasing amounts of PE. Fig. 9B shows that the aptamer could be used to purify conveniently detectable amounts of HPPD when incubated with different amounts of HPPD and in the presence of PE. By contrast, the protein band assigned to HPPD was not visible when negative controls (beads only and N47α immobilised on beads) were used.

This example illustrates that anti-interaction partner aptamers can be used to purify significant amounts of interaction partners under near-physiological conditions, which could enable researchers to characterise said interaction partner.

### Example 9: Using tightly-binding, immobilised aptamers to pull down and characterise the interaction partner present in protein extract

For a realistic proof of principle scenario representing attempts to identify an unknown interaction partner, biotinylated aptamer S116D-C8 was immobilised and used in attempts to pull down endogenous HPPD in protein extract, *i.e*. no recombinant protein was added. Again, appropriate controls such as N47α as a negative control and S116D-C8 together with recombinant HPPD were included. Indeed, a protein band was visually detectable if the aptamer S116D-C8 was used as the affinity ligand and protein extract only as the source of HPPD.

Analysis of the protein band using mass spectrometry and subsequently subjecting the results to data base analysis resulted in a number of hits that rank as follows:
1. Ribulose 1,5-bisphosphate carboxylase (RubisCo)
2. 4-Hydroxyphenylpyruvate dioxygenase (HPPD)
3. Disease resistance protein

Considering that RubisCo is an abundant protein in plant leaves (as demonstrated by its strong protein band in protein extract, for example in Fig. 10). and, at a molecular weight of approximately 53 kDa runs extremely close to HPPD (molecular weight of approximately 52 kDa), this is an extremely satisfactory result, as it shows that the interaction partner can be identified using authentic biological source material and the method according to the present invention. If these three hits were the result of an attempt to identify the interaction partner of the active agent sulcotrione, the three proteins could now be cloned, expressed, purified and used in binding assays, which would vigorously identify HPPD as the interaction partner of interest.

In summary, these examples demonstrate that it is possible (i) to obtain aptamers binding specifically and with high affinity to the binding site of a known active agent on an unknown interaction partner, (ii) to chemically modify such aptamers without abolishing their ability to bind the interaction partner of interest and (iii) to isolate and characterise said interaction partner.

The features of the present invention disclosed in the specification, the sequence listing, the claims and/or the drawings may both separately and in any combination thereof be material for realizing the invention in various forms thereof.

## Claims

1. A method for identifying an interaction partner of an active agent, comprising the following steps:
(a) providing an element library comprising a plurality of elements,
(b) contacting the element library with a preparation comprising an interaction partner under conditions allowing for formation of a complex consisting of a binding element of the element library binding to the interaction partner and the interaction partner, whereupon a complex comprising the binding element and the interaction partner is formed,
(c) removing non-binding elements of the element library from the complex formed in step (b),
(d) contacting the complex formed in step (b) with the active agent, whereby the binding element is released from said complex,
(e) contacting the binding element released in step (d) with a preparation comprising the interaction partner, whereupon a complex is formed comprising the binding element released in step (d) and the interaction partner, and
(f) characterising the interaction partner.

2. The method according to claim 1, comprising as a further step (d') the isolation of the binding element released in step (d).

3. The method according to claim 1, comprising as a further step (d") the amplification of the element released in step (d).

4. The method according to claims 1 to 3, wherein the steps (a) to (d), (a) to (d') or (a) to (d") are repeated.

5. The method according to any of claims 1 to 4, wherein step (d) comprises the unspecific or specific elution of the binding element.

6. The method according to any of claims 1 to 5, wherein the method is for identifying two or more interaction partners.

7. The method according to any of claims 1 to 6, wherein the elements are ribonucleic acids.

8. The method according to any of claims 1 to 6, wherein the elements are deoxyribonucleic acids.

9. The method according to any of claims 1 to 6, wherein the elements are peptides.

10. The method according to any of claims 1 to 9, wherein any of the preparations comprising the interaction partner is selected from the group consisiting of a proteome of an organism or a fraction thereof, a cellular extract of an organism, an extracellular extract of an organism, a subcellular extract of an organism and an extract of a whole organism.

11. The method according to any of claims 1 to 10, wherein the binding element released in step (d) is immobilised on a support prior to step (e).

12. The method according to any of claims 1 to 11, wherein the active agent is selected from the group consisting of pharmaceutically active agents, agrochemically active agents, therapeutically active agents, diagnostically active agents, synergists, veterinary pharmaceuticals, animal and human nutrition-enhancing compounds comprising nutraceuticals, and plant protection agents, and a respective precursor of each thereof.

13. The method according to any of claims 1 to 12, wherein the interaction partner is **characterised by** a technique selected from the group consisting of mass spectrometry, sequencing techniques, binding assays, enzyme activity assays, electrophoresis and immunological methods.

14. The method according to claim 12, wherein the plant protection agent is selected from the group consisting of herbicides, fungicides, active agents against oomycetes, nematocides, insecticides, safeners, combinations of safeners and plant protection agents.

15. The method according to claim 12, wherein the veterinary pharmaceuticals are selected from the group consisting of antiviral compounds, antibiotics and anti-parasite drugs.

16. The method according to any of claims 1 to 15, wherein the interaction partner is selected from the group consisting of peptides, polypeptides, proteins, nucleic acids, lipids, carbohydrates, or any combination thereof.

17. The method according to any of claims 1 to 13, wherein the interaction partner is a protein, whereby the protein is preferably selected from the group consisting of enzymes, receptors and transcription factors.

18. The method according to any of claims 1 to 17, wherein the various elements of the element library comprise a variable moiety and a constant moiety, whereby the constant moiety is the same for the majority, preferably all of the elements of the element library.

19. The method according to claim 18, wherein the element library consists of nucleic acids and the constant moiety consists of a 3'-terminal or 5'-terminal stretch of nucleotides.

20. The method according to claim 19, wherein the constant moiety consists of from about 10 to about 30 nucleotides.

21. The method according to any of claims 18 to 20, wherein the element library consists of nucleic acids, whereby the variable moiety consists of from about 8 to 120 nucleotides.

22. The method according to claim 18, wherein the element library consists of peptides and the constant moiety consists of an N-terminal or C-terminal stretch of amino acids.

23. The method according to any of claims 1 to 22, wherein the step of removing non-binding elements as of step (c), the step of isolating the binding elements as of step (d') and/or the step of amplifying the elements as of step (d") make use of the constant moiety of the elements of the element library.

24. The method according to any of claims 1 to 23, wherein the elements are nucleic acids and wherein the amplification is performed by reverse transcription, PCR, RNA transcription or isothermal amplification.

25. The method according to any of claims 1 to 23, wherein the elements are peptides expressed by a phage display technique and the amplification is performed by amplifying the phages expressing the peptides.

26. The method according to any of claims 10 to 25, wherein the organism is selected from the group consisting of humans, prokaryotes, fungi, animals and plants.

27. The method according to any of claims 11 to 26, wherein the immobilisation of the binding element is mediated by the constant moiety of the element.

28. An interaction partner identifiable by a method according to any of claims 1 to 27.

29. A method for determining the mode of action of an active agent, comprising the steps of the method according to any of claims 1 to 27.


SEQUENCE LISTING

<110>  Bayer Crop Science AG

<120>  Method for identifying the interaction partner of an active agent

<130>  BCS 07-1028

<160>  5

<170>  PatentIn version 3.1

<210>  1

<211>  116

<212>  DNA

<213>  Artificial sequence

<220>

<223>  Chemically synthesised

<220>

<221>  misc_feature

<222>  (48)..(94)

<223>  nucleotide = a, t, g, c

<400>  1
aattctaata cgactcacta tagggagagg agggagatag atatcaannn nnnnnnnnnn      60

nnnnnnnnnn nnnnnnnnnn nnnnnnnnnn nnnnagtttg tcctcacggt ggatgg         116

<210>  2

<211>  95

<212>  RNA

<213>  Artificial sequence

<220>

<223>  Chemically synthesised

<400>  2
gggagaggag ggagauagau aucaacaaug acuagccgug uaaguggucu cguacauccg      60

aaaaagacga cccaguuugu ccucacggug gaugg                                95

<210>  3

<211>  93

<212>  RNA

<213>  Artificial Sequence

<220>

<223>  Chemically synthesised

<400>  3
gggagaggag ggagauagau aucaauaucg ccacguuacg aguaccaagc uuuauuuccg      60

cagcccgaua gaguuugucc ucacggugga ugg                                  93

<210>  4

<211>  94

<212>  RNA

<213>  Artificial sequence

<220>

<223>  Chemically synthesised

<400>  4
gggagaggag ggagauagau aucaaguaac aaaugauacu uuccaagcau aauucauucg      60

cagcccgaau uaaguuuguc cucacggugg augg                                  94

<210>  5

<211>  94

<212>  RNA

<213>  Artificial sequence

<220>

<223>  Chemically synthesised

<400>  5
gggagaggag ggagauagau aucaauaaag ucggugacac cacgcaucca aauaccggua      60

cgcggaacua auaguuuguc cucacggugg augg                                  94